# EUROPEAN PATENT SPECIFICATION

(11) **EP 1 945 610 B1**
(45) Date of publication and mention of the grant of the patent: **20.01.2010**
(21) Application number: 06805702.5
(22) Date of filing: 13.09.2006
(51) Int. Cl.: C07C 403/14, C07D 301/26, C07D 303/48

(54) **PROCESS FOR THE PREPARATION OF AN EPOXY COMPOUND AND AN ALDEHYDE**
VERFAHREN ZUR HERSTELLUNG EINER EPOXYVERBINDUNG UND EINES ALDEHYDS
PROCEDE POUR LA PREPARATION D'UN COMPOSE EPOXY ET D'UN ALDEHYDE

(30) Priority: 16.09.2005 EP 05020206
(43) Date of publication of application: 23.07.2008
(73) Proprietor: DSM IP Assets B.V., 6411 TE Heerlen (NL)
(72) Inventor: STANGL, Jochen, 79664 Wehr (DE); STRITT, Claude, CH-4056 Basle (CH)
(74) Representative: Pressner, Dietmar
(86) International application number: PCT/EP2006/008894
(87) International publication number: WO 2007/031286

(56) References cited:
- EP-A- 0 371 264
- US-A- 2 987 550

## Description

The invention relates to a process comprising a condensation step wherein a starting aldehyde or ketone is made to react with an ester of an α-haloacid to form an epoxy compound, in particular a glycidic ester.

An example of such a process is a process comprising the known Darzens-type condensation reaction, discussed in for example US 2,987,550. In US 2,987,550 the Darzens preparation is disclosed as being a part of the preparation of the so-called 'C₁₄ aldehyde'. In US 2,987,550 and also within the context of the present invention the term 'C₁₄ aldehyde' refers to 2-methyl-4-(2,6,6-trimethyl-1-cyclohexene-1-yl)-2-butenal, an important intermediate in various processes such as the preparation of vitamin A, having the structural formula (I):

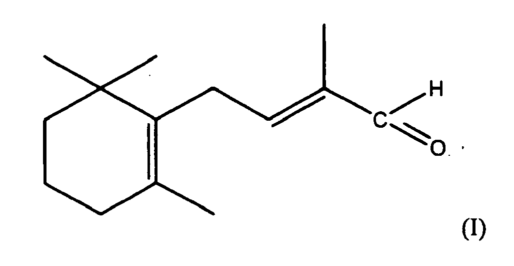

In the known process, the Darzens condensation is carried out by creating a reaction mixture of β-ionone and an excess of a lower alkyl chloroacetate, e.g. methyl or ethyl chloroacetate. As is known, the term β-ionone refers to 4-(2,6,6-trimethyl-1-cyclohexene-1-yl)-3-buten-2-one, a compound having formula (II):

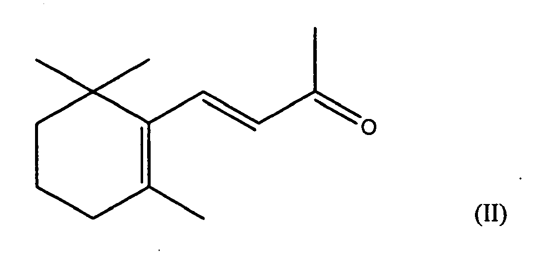

During the mixing of the β-ionone with the alkyl chloroacetate in the known process, an essentially anhydrous diluent is added; the diluent is in the known process chosen from the group consisting of dimethylformamide, pyridine, α-, β-, or γ-picoline, dimethylacetamide and N-ethylacetamide. This group of diluents comprises dipolar aprotic diluents. A dry, alcohol-free alkali metal alkoxide is also added to the reaction mixture. The reaction mixture is subsequently treated with cold methanolic sodium hydroxide at a low temperature, followed by recovery of the C₁₄ aldehyde. Recovery is in the known process typically done by pouring the reaction mixture into water, followed by extraction by an immiscible solvent such as hexane.

The known process has as disadvantage that a high amount of the compound acting as diluent should be used in order to achieve a high yield. As illustration of this, reference is made to examples I and III of US 2,987,550, where a reduction of the molar ratio of the diluent dimethylformamide to β-ionone from about 2.2 in example I to about 1.1 in example III leads to a reduction of yield from 84.8% to 78%. Furthermore, reference is made to Example V. In this example, a yield of 84.6 % was achieved; however, about 5 moles of the diluent pyridine was used per 1.11 mole of β-ionone, i.e. a ratio of about 4.5. Similar excess ratios of diluents are found in the other examples.

It is the objective of the present invention to reduce the disadvantage of the known process.

The said objective is achieved in that the condensation step is carried out in the presence of a dipolar aprotic compound selected from the group consisting of 1-methyl-2-pyrrolidone (NMP), hexamethylfosforic acidtriamid (HMPT), dimethylsulfoxide (DMSO), and urea derivatives of the following formula (III): 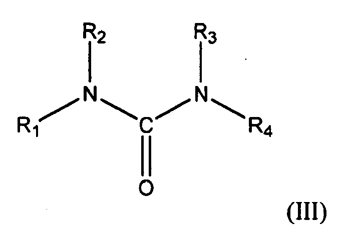 wherein R₁, R₂, R₃ and R₄ may each independently be H or a C₁-C₄ alkyl group and whereby R₂ and R₃ may together form a heterocyclic group.

The process according to the invention has the advantage that very high selectivities and yields can be achieved without having to use high amounts - in relation to the starting aldehyde or ketone - of a further compound such as a compound acting as diluent.

A further advantage of the process according to the invention is that also the molar excess of the ester of α-haloacid - as compared to the starting aldehyde or ketone - can be reduced compared to the known process.

Yet another advantage of the process according to the invention is that the dipolar aprotic compound selected according to the invention can have fewer or even no health- or environmental objections, and fewer objections regarding unpleasant odours, which is important because compounds such as the C₁₄ aldehyde are a.o. used in the preparation of perfumes.

In CN 1348947, a process for the production of C₁₄ aldehyde from β-ionone via a Darzens condensation and using a solvent is disclosed. According to CN 1348947, the solvent should be a compound according to the formula (IV) as given below.

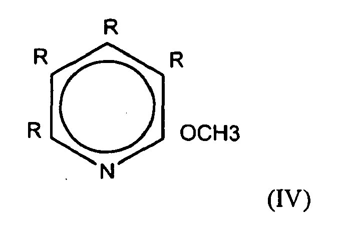

The process according to the invention comprises a condensation step. In this step, a starting aldehyde or a starting ketone are present as raw material(s). Aldhydes and ketones are as such known. In the process according to the invention, a wide variety of aldehydes or ketones may be used; preferably, they are aromatic. An example of a starting ketone is β-ionone, the compound according to formula (II). Further examples of preferred starting aldehydes or starting ketones are represented by formula (VII): 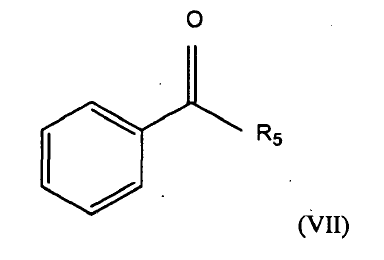 wherein R₅ is preferably H - so that the compound according to formula (VII) is benzaldehyde - or CH₃ - so that the compound according to formula (VII) is acetophenone. R₅ may thus be H or CH₃ but also a C₂ - C₆ alkyl group.

In the condensation step, an ester of an α-haloacid is also present. Preferably, the ester of an α-haloacid is methylchloroacetate, ethylchloroacetate, propylchloroacetate or butylchloroacetate; most preferably, the ester of an α-haloacid is methylchloroacetate.

According to the invention, the starting aldehyde or ketone and the ester of an α-haloacid are made to react with each other. This should be done in such a fashion - preferably by carrying out the reaction in the presence of a base - that an epoxy compound is formed. As is known, epoxy compounds are characterised by the oxygen-containing three-membered ring as part of the compound. In particular, glycidic esters can be formed in this fashion. One known method of achieving this is via the known Darzens type of condensation reaction. As is known, in a Darzens condensation reaction the starting aldehyde or ketone is brought together with the ester of an α-haloacid; in order to make these react with each other, an alkali metal alkoxide (preferably dry and alcohol-free) is added as the base; in this fashion, the glycidic ester is formed. As alkali metal alkoxide, sodium methylate is preferred. Generally speaking, the 'alkoxide part' of the alkali metal alkoxide is preferably chosen so that it matches the 'ester part' of the ester of an α-haloacid (e.g., alkali metal *methylate* and the *methyl* ester of an α-haloacid such as chloroacetic acid); this has the advantage that the creation of a mixture of different alcohols is avoided.

In a preferred embodiment of the condensation step, β-ionone is the starting ketone, methylchloroacetate is the ester of an α-haloacid and sodium methylate is the alkali metal alkoxide. The glycidic ester thus formed has formula (V):

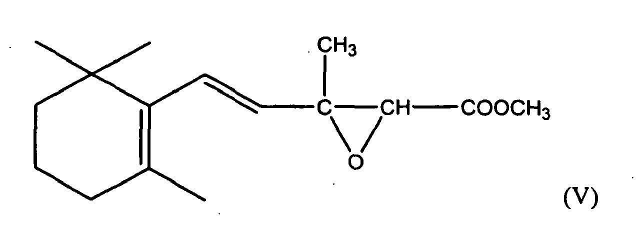

In another preferred embodiment of the condensation step, benzaldehyde - i.e. the compound according to formula (VII) with R₅ being H - is the starting aldehyde and a lower alkyl- (e.g. methyl- or ethyl-) chloroacetate is the ester of an α-haloacid. The glycidic ester thus formed is ethyl 3-phenylglycidate, a compound according to formula (VIII):

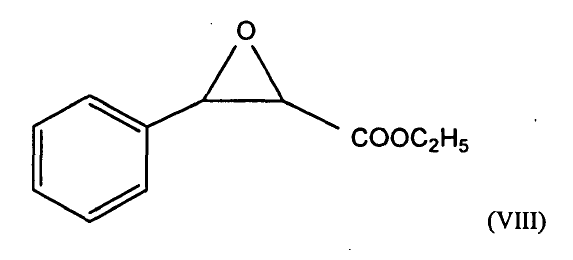

In a further preferred embodiment of the condensation step, acetophenone - i.e. the compound according to formula (VII) with R₅ being CH₃ - is the starting aldehyde and a lower alkyl (e.g. methyl- or ethyl-) chloroacetate is the ester of an α-haloacid. The glycidic ester thus formed is ethyl 3-methyl-3-phenylglycidate, a compound according to formula (IX):

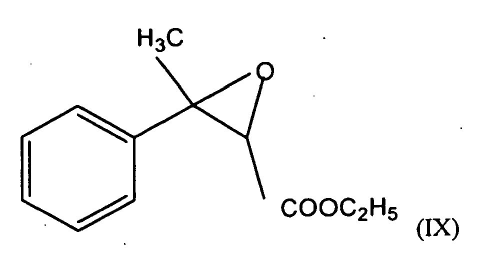

The compounds according to formulas (VIII) and (IX) are as such known, from e.g. section 2.7.3 of the Flavors and Fragrances chapter of Ullmann's Encyclopedia of Industrial Chemistry, DOI 10.1002/14356007.a11 141, posted online on 15.01.2003.

According to the invention, the condensation step is carried out in the presence of a further compound, not being the starting aldehyde or ketone, the ester of an α-haloacid or the alkali metal alkoxide. In contrast to the known process where the said further compound is chosen from the group as detailed in the discussion above on the known process, the further compound is a dipolar aprotic compound or mixture of compounds selected from the group consisting of 1-methyl-2-pyrrolidone (NMP), hexamethylfosforic acidtriamid (HMPT), dimethylsulfoxide (DMSO), and urea derivatives of the following formula: 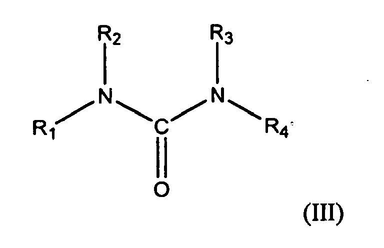 wherein R₁, R₂, R₃ and R₄ may each independently be H or a C₁-C₄ alkyl group and whereby R₂ and R₃ may together form a heterocyclic group. Without committing to scientific theory, it is thought that the further compound according to the invention does not significantly react during the condensation step, but rather may act as diluent and/or as solvent and/or as heat transfer medium and/or in yet another fashion.

As is known, the compound 1-methyl-2-pynolidone (NMP) has formula (VI).

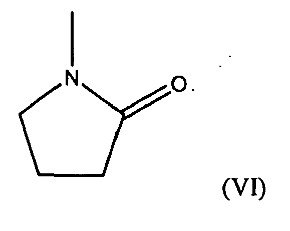

In formula (III), it is preferred that R₁, R₂, R₃ and R₄ are each independently H or a methyl or an ethyl group. In another embodiment, it is preferred that R₁, R₂, R₃ and R₄ are identical. In another class of preferred embodiments of the compound according to formula (III), R₂ and R₃ are such that compound (III) comprises a heterocyclic ring. As is known, the term heterocyclic ring indicates a ring structure wherein the ring-forming atoms are not all carbon. Since R₂ and R₃ will in the case of ring-forming only contribute carbon atoms as ring-forming atoms, this implies that within the context of the present invention the two nitrogen atoms are comprised within the ring structure. If the compound according to formula (III) has a heterocyclic ring, then this ring is preferably a 5- or 6-ring. Preferred examples of compounds according to formula (III) are urea, 1,3-dimethyltetrahydro-2(1H)-pyrimidinone (DMPU) or 1,1,3,3-tetramethylurea (TMU).

The dipolar aprotic compound as selected according to the invention does not need to be in liquid form, in particular not if the starting aldehyde or ketone and/or the ester of an α-haloacid are in liquid form; a liquid form is, nevertheless, preferred. Most preferably, the condensation step is carried out in the presence of NMP.

It is an advantage of the condensation step according to the invention that high yields towards the epoxy compound such as a glycidic ester may be achieved with the utilisation of a relatively limited amount of the further compound such as NMP. It is preferred that the molar ratio in the condensation step between the dipolar aprotic compound - as selected according to the invention - and the starting aldehyde or ketone lies between 0.05 and 2. The said molar ratio is preferably higher than 0.05, so that the desired effect of yield enhancement is clearly noticeable. More preferably, the said ratio is at least 0.08, 0.10, 0.12, or 0.15. The said molar ratio is preferably at most 2.00 so as to reap the maximum economical benefit from the condensation step according to the invention. More preferably, the said molar ratio is at most 1.90, 1.80, 1.75, 1.70, or 1.65.

It is an advantage of the condensation step according to the invention that the molar excess of the ester of an α-haloacid - as compared to the starting aldehyde or ketone - can be reduced compared to the known process. This is particularly advantageous since this can be - and preferably is - combined with a low molar ratio between the dipolar aprotic compound as selected according to the invention and the starting aldehyde or ketone, as detailed above. Preferably, the molar ratio between the ester of an α-haloacid and the starting aldehyde or ketone lies between 1.00 and 2.00. More preferably, the said ratio is at least 1.05, 1.10, or 1.15, and at most 1.90, 1.80, or 1.70.

It is a further advantage of the condensation step according to the invention that the molar excess of the base such as the alkali metal alkoxide - as compared to the starting aldehyde or ketone - can be reduced compared to the known process. This is particularly advantageous since this can be - and preferably is - combined with a low molar ratio between the dipolar aprotic compound as selected according to the invention and the starting aldehyde or ketone, as detailed above. Even more advantageous is the preferred embodiment of also combining the low amount of alkali metal alkoxide with a low amount of an ester of an α-haloacid, according to the ranges as detailed above. Preferably, the molar ratio between the alkali metal alkoxide and the starting aldehyde or ketone lies between 1.00 and 2.00. More preferably, the said ratio is at least 1.05, 1.10, or 1.15, and at most 1.90, 1.80, or 1.70.

The condensation step according to the invention may typically be carried out at atmospheric pressures; there is usually not a ground to operate at reduced or increased pressures, although this is certainly possible should the characteristics of one of the compounds present require this according to the knowledge of those skilled in the art. It was found furthermore that it is often beneficial to carry out the condensation step according to the invention in a temperature range lying between -20°C and +10°C, as in this range both selectivity and yield are good. Within this range, the selectivity towards epoxy compound, in particular glycidic ester formation tends to increase as the temperature decreases. More preferably, the said temperature range goes from -15°C to +5°C.

It is preferred that in the condensation step according to the invention the compounds designated as diluents or solvents in the known processes such as those of US 2,987,550 or CN 1348947 are present in only limited amounts, i.e. less than 50 wt.%, 40, 30, or even less than 10% compared to the amount of the dipolar aprotic compound selected according to the invention. More preferably, the said compounds according to the known processes are essentially absent.

In practice and preferred according to the present invention, the condensation step is followed by a saponification step. In the saponification step, the principle of which is as such known, the epoxy compound such as a glycidic ester as formed in the condensation step is saponified. The said saponification may be achieved in a known fashion, e.g. by bringing the glycidic ester in contact with sodium hydroxide, whereby the said sodium hydroxide is typically anhydrous, and often dissolved in an alcohol such as methanol. According to a preferred embodiment according to the invention, the saponification step is done on the glycidic ester according to formula (V).

In practice and also preferred according to the present invention, the saponification step is followed by a hydrolysis step. In this step, the saponified epoxy compound (e.g., the saponified glycidic ester) is brought into contact with an aqueous phase; this leads to decarboxylation of the saponified epoxy compound or glycidic ester, whereby an aldehyde or ketone is formed. According to a preferred embodiment according to the invention, the hydrolysis step is done on the glycidic ester according to formula (V) as saponified in the saponification step; the resulting aldehyde is then the C₁₄ aldehyde according to formula (I).

Upon completion of the hydrolysis step, an aqueous system is present comprising water, the aldehyde or ketone as formed, and also the dipolar aprotic compound as selected according to the invention. The aldehyde or ketone as formed may be recovered with methods known to the skilled person. An example of such a method is an extraction step in which the aqueous system is brought in contact with an extracting agent, said extracting agent being limitedly or essentially non-miscible with water, and chosen such that the aldehyde or ketone migrates preferentially into the extracting agent. Examples of extracting agents are alkanes such as hexane.

The invention will be illustrated by means of the following examples, without being limited thereto.

### Example 1

β-ionone (212.7 g, 97% purity, 1.07 moles), methylchloroacetate (146.4 g, 1.35 moles) and NMP (75 g, 0.76 moles) were put into a stirred reactor and cooled to 0°C. Sodium methylate (87.6 g, 1.62 moles) was then dosed, leading to the reaction to the corresponding glycid ester. After the sodium methylate had been added, the reactor contents were brought to 20°C and remained there for 20 minutes. In this Example, the molar ratio of NMP to β-ionone was 0.71; the molar ratio of methlychloroacetate to β-ionone was 1.23; the molar ratio of sodium methylate to β-ionone was 1.51.

Subsequent to the formation of the glycidic ester, saponification was achieved through addition of sodium hydroxide, whereby the temperature rose to 35°C. The reaction mixture was then transferred to a second stirred vessel; in the said second stirred vessel, the hydrolysis reaction to the C₁₄ aldehyde was done by means of addition of water. Then, hexane was added to the reaction mixture - while being stirred. This lead to the extraction of the C₁₄ aldehyde from the aqueous mixture into the hexane phase. The hexane phase was separated from the aqueous phase and acidified through the addition of some aqueous acetic acid. The C₁₄ aldehyde was then isolated through evaporation of the hexane under vacuum followed by distillation. A total of 205.6 g of the C₁₄ aldehyde was recovered, corresponding to a yield of 89.4% (calculated from the raw material (β-ionone).

### Examples 2 - 5

The procedure of Example 1 was repeated, the only difference being the amount of NMP that was added. The results are given in the table below.

| Example | Amount of NMP (g) | Molar ratio NMP/β-ionone (-) | Yield of C₁₄ aldehyde (%) |
|---|---|---|---|
| 2 | 15 | 0.14 | 84.4 |
| 3 | 45 | 0.42 | 88.5 |
| 4 | 120 | 1.13 | 88.9 |
| 5 | 165 | 1.56 | 85.5 |

### Examples 6 - 10

The procedure of Example 1 was repeated; however, instead of NMP, DMPU was added in various amounts. The results are given in the table below.

| Example | Amount of DMPU (g) | Molar ratio DMPU/β-ionone (-) | Yield of C₁₄ aldehyde (%) |
|---|---|---|---|
| 6 | 15 | 0.11 | 86.0 |
| 7 | 45 | 0.33 | 89.0 |
| 8 | 75 | 0.55 | 87.6 |
| 9 | 120 | 0.88 | 86.0 |
| 10 | 165 | 1.20 | 84.4 |

### Examples 11-15

The procedure of Example 1 was repeated; however, instead of NMP, TMU was added in various amounts. The results are given in the table below.

| Example | Amount of TMU (g) | Molar ratio TMU/ β-ionone (-) | Yield of C₁₄ aldehyde (%) |
|---|---|---|---|
| 11 | 15 | 0.13 | 84.7 |
| 12 | 45 | 0.40 | 88.8 |
| 13 | 75 | 0.66 | 90.0 |
| 14 | 120 | 1.06 | 90.5 |
| 15 | 165 | 1.46 | 90.4 |

## Claims

1. Process comprising a condensation step wherein a starting aldehyde or ketone is made to react with an ester of an α-haloacid to form an epoxy compound, whereby the reaction is carried out in the presence of a dipolar aprotic compound selected from the group consisting of 1-methyl-2-pyrrolidone (NMP), hexamethylphosphorous triamide (HMPT), dimethylsulfoxide (DMSO), and urea derivatives of the following formula: 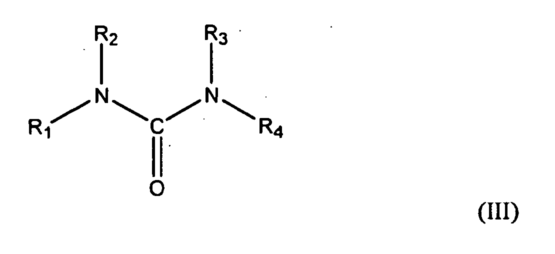 wherein R₁, R₂, R₃ and R₄ may each independently be H or a C₁-C₄ alkyl group and whereby R₂ and R₃ may together form a heterocyclic group.

2. Process according to claim 1, wherein the condensation step is carried out in the presence of a base.

3. Process according to claim 2, wherein the base is an alkali metal alkoxide.

4. Process according to any one of claims 1 - 3, wherein the epoxy compound is a glycidic ester, and wherein the process further comprises a saponification step, wherein the glycidic ester is saponified, and a hydrolysis step, wherein the saponified glycidic ester is decarboxylated to form an aldehyde.

5. Process according to any one of claims 1 - 4, wherein the raw materials for the condensation step comprise β-ionone.

6. Process according to claim 4, wherein the raw materials for the condensation step comprise β-ionone, methylchloroacetate, sodium methylate and 1-methyl-2-pyrrolidon (NMP), and whereby in the hydrolysis step the C₁₄ aldehyde 2-methyl-4-(2,6,6-trimethyl-1-cyclohexene-1-yl)-2-butenal is formed.

7. Process according to any one of claims 1 - 3, wherein the raw materials for the condensation step comprise benzaldehyde and/or acetophenone.

8. Process according to any one of claims 1 - 7, wherein the molar ratio in the condensation step between the dipolar aprotic compound and the starting aldehyde or ketone lies between 0.05 and 2.

9. Process according to any one of claims 1 - 8, wherein the molar ratio in the condensation step between the ester of an α-haloacid and the starting aldehyde or ketone lies between 1.0 and 2.0.

10. Process according to any one of claims 3 - 9, wherein the molar ratio in the condensation step between the alkali metal alkoxide and the starting aldehyde or ketone lies between 1.0 and 2.0.

11. Process according to claim 1, wherein the dipolar aprotic compound is according to formula (III) and is 1,3-dimethyltetrahydro-2(1H)-pyrimidinone (DMPU) or 1,1,3,3-tetramethylurea (TMU).

12. Process for the preparation of vitamin A or an ester of vitamin A, **characterised in that** the process comprises the process according to anyone of claims 1 - 8.

## Patentansprüche

1. Verfahren mit einem Kondensationsschritt, bei dem ein Ausgangsaldehyd oder -keton mit einem Ester einer α-Halogensäure zu einer Epoxidverbindung umgesetzt wird, wobei die Umsetzung in Gegenwart einer dipolaren aprotischen Verbindung aus der Gruppe bestehend aus 1-Methyl-2-pyrrolidon (NMP), Hexamethylphosphorsäuretriamid (HMPT), Dimethylsulfoxid (DMSO) und Harnstoffderivaten der folgenden Formel: 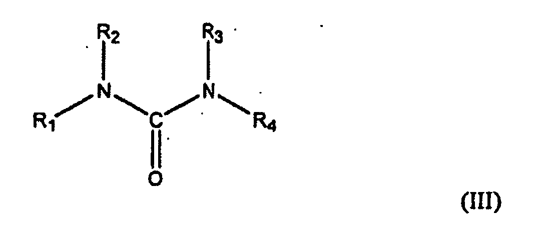 worin R₁, R₂, R₃ und R₄ jeweils unabhängig voneinander für H oder eine C₁-C₄-Alkylgruppe stehen können und R₂ und R₃ zusammen eine heterocyclische Gruppe bilden können, durchgeführt wird.

2. Verfahren nach Anspruch 1, bei dem der Kondensationsschritt in Gegenwart einer Base durchgeführt wird.

3. Verfahren nach Anspruch 2, bei dem es sich bei der Base um ein Alkalimetallalkoxid handelt.

4. Verfahren nach einem der Ansprüche 1 - 3, bei dem es sich bei der Epoxidverbindung um einen Glycidester handelt, und bei dem das Verfahren ferner einen Verseifungsschritt, bei dem der Glycidester verseift wird, und einen Hydrolyseschritt, bei dem der verseifte Glycidester zu einem Aldehyd decarboxyliert wird, umfaßt.

5. Verfahren nach einem der Ansprüche 1 - 4, bei dem die Ausgangsstoffe für den Kondensationsschritt β-Ionon umfassen.

6. Verfahren nach Anspruch 4, bei dem die Ausgangsstoffe für den Kondensationsschritt β-Ionon, Chloressigsäuremethylester, Natriummethylat und 1-Methyl-2-pyrrolidon (NMP) umfassen, und wobei im Hydrolyseschritt der C₁₄-Aldehyd 2-Methyl-4-(2,6,6-trimethyl-1-cyclohexen-1-yl)-2-butenal gebildet wird.

7. Verfahren nach einem der Ansprüche 1 - 3, bei dem die Ausgangsstoffe für den Kondensationsschritt Benzaldehyd und/oder Acetophenon umfassen.

8. Verfahren nach einem der Ansprüche 1 - 7, bei dem das Molverhältnis im Kondensationsschritt zwischen der dipolaren aprotischen Verbindung und dem Ausgangsaldehyd oder -keton zwischen 0,05 und 0,2 liegt.

9. Verfahren nach einem der Ansprüche 1 - 8, bei dem das Molverhältnis im Kondensationsschritt zwischen dem Ester einer α-Halogensäure und dem Ausgangsaldehyd oder -keton zwischen 1,0 und 2,0 liegt.

10. Verfahren nach einem der Ansprüche 3 - 9, bei dem das Molverhältnis im Kondensationsschritt zwischen dem Alkalimetallalkoxid und dem Ausgangsaldehyd oder -keton zwischen 1,0 und 2,0 liegt.

11. Verfahren nach Anspruch 1, bei dem die dipolare aprotische Verbindung der Formel (III) entspricht und 1,3-Dimethyltetrahydro-2(1H)-pyrimidinon (DMPU) oder 1,1,3,3-Tetramethylharnstoff (TMU) ist.

12. Verfahren zur Herstellung von Vitamin A oder einem Ester von Vitamin A, **dadurch gekennzeichnet, daß** das Verfahren das Verfahren nach einem der Ansprüche 1 - 8 umfaßt.

## Revendications

1. Procédé comprenant une étape de condensation dans laquelle on fait réagir un aldéhyde ou une cétone de départ avec un ester d'un α-haloacide pour former un composé époxy, la réaction étant effectuée en présence d'un composé aprotique dipolaire choisi dans le groupe constitué de 1-méthyl-2-pyrrolidone (NMP), de triamide hexaméthylphosphorique (HMPT), de diméthylsulfoxyde (DMSO) et de dérivés d'urée de la formule suivante : 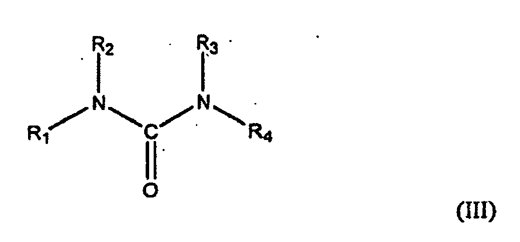 dans laquelle R₁, R₂, R₃ et R₄ peuvent être chacun indépendamment H ou un groupement alkyle en C₁-C₄ et R₂ et R₃ pouvant former ensemble un groupement hétérocyclique.

2. Procédé selon la revendication 1, **caractérisé en ce que** l'étape de condensation est effectuée en présence d'une base.

3. Procédé selon la revendication 2, **caractérisé en ce que** la base est un alcoxyde de métal alcalin.

4. Procédé selon l'une quelconque des revendications 1 à 3, **caractérisé en ce que** le composé époxy est un ester glycidique, et **en ce que** le procédé comprend en outre une étape de saponification, dans laquelle l'ester glycidique est saponifié, et une étape d'hydrolyse, dans laquelle l'ester glycidique saponifié est décarboxylé pour former un aldéhyde.

5. Procédé selon l'une quelconque des revendications 1 à 4, **caractérisé en ce que** les matières premières pour l'étape de condensation comprennent la β-ionone.

6. Procédé selon la revendication 4, **caractérisé en ce que** les matières premières pour l'étape de condensation comprennent la β-ionone, le chloroacétate de méthyle, le méthylate de sodium et la 1-méthyl-2-pyrrolidone (NMP) et **en ce que** dans l'étape d'hydrolyse, l'aldéhyde en C₁₄ 2-méthyl-4-(2,6,6-triméthyl-1-cyclohexène-1-yl)-2-butenal est formé.

7. Procédé selon l'une quelconque des revendications 1 à 3, **caractérisé en ce que** les matières premières pour l'étape de condensation comprennent le benzaldéhyde et/ou l'acétophénone.

8. Procédé selon l'une quelconque des revendications 1 à 7, **caractérisé en ce que** le rapport molaire dans l'étape de condensation entre le composé aprotique dipolaire et l'aldéhyde ou la cétone de départ est compris entre 0,05 et 2.

9. Procédé selon l'une quelconque des revendications 1 à 8, **caractérisé en ce que** le rapport molaire dans l'étape de condensation entre l'ester d'un α-haloacide et l'aldéhyde ou la cétone de départ est compris entre 1,0 et 2,0.

10. Procédé selon l'une quelconque des revendications 3 à 9, **caractérisé en ce que** le rapport molaire dans l'étape de condensation entre l'alcoxyde de métal alcalin et l'aldéhyde ou la cétone de départ est compris entre 1,0 et 2,0.

11. Procédé selon la revendication 1, **caractérisé en ce que** le composé aprotique dipolaire est selon la formule (III) et est la 1,3-diméthyltétrahydro-2(1H)-pyrimidinone (DMPU) ou la 1,1,3,3-tétraméthylurée (TMU) .

12. Procédé de préparation de la vitamine A ou d'un ester de la vitamine A, **caractérisé en ce que** le procédé comprend le procédé selon l'une quelconque des revendications 1 à 8.
